# EUROPÄISCHE PATENTANMELDUNG

(11) **EP 1 429 125 A1**
(43) Veröffentlichungstag der Anmeldung: **16.06.2004**
(21) Anmeldenummer: 03022203.8
(22) Anmeldetag: 30.09.2003
(51) Int. Cl.: G01F 23/26, G01N 33/28

(54) **Kapazitiver Materialsensor**

(30) Priorität: 13.12.2002 DE 10258418
(71) Anmelder: Beru AG, 71636 Ludwigsburg (DE)
(72) Erfinder: Marto, Arno, 71263 Weil der Stadt (DE); Bantzhaff, Ralf, 74343 Sachsenheim (DE)
(74) Vertreter: Pohlmann, Eckart, Dipl.-Phys.

(57) **Zusammenfassung**

Mediensensor zum Erfassen eines Mediums und/oder zum Bestimmen der Beschaffenheit eines Mediums mit einem Gehäuse (1), an dem ein Kondensator angeordnet ist, zwischen dessen Elektroden (3, 4) ein Medium eingeführt werden kann. Der Kondensatorelektroden sind koaxiale Hohlkörper, die ein homogenes elektrisches Feld erzeugen, und vorzugsweise koaxiale zylindrische Elektroden.

## Beschreibung

Die Erfindung betrifft einen Mediensensor zum Erfassen eines Mediums und/oder zum Bestimmen der Beschaffenheit eines Mediums mit einem Gehäuse, in dem ein Kondensator angeordnet ist, zwischen dessen Elektroden ein Medium eingeführt werden kann.

Derartige kapazitive Mediensensoren werden auf dem Gebiet der Kraftfahrzeugtechnik beispielsweise zur Kraftstofferkennung, zur Ermittlung einer Medienqualität, insbesondere der Ölqualität und zur Erkennung und Erfassung des Auftretens von Wasser benutzt.

Aus der DE 100 60 419 C2 ist ein Mediensensor insbesondere ein Wassersensor bekannt, der in Form eines elektrischen Widerstandes ausgebildet ist, dessen Ohmscher Widerstand von dem zu erfassenden Medium abhängt. Ein derartiger Medien- oder Wassersensor kann Medien über die verschiedenen Leitfähigkeiten unterscheiden und dient insbesondere zum Schutz des Einspritzsystems eines Kraftfahrzeuges vor dem Eindringen von Wasser.

Ein derartiger Sensor ist jedoch nur in der Lage das Auftreten eines bestimmten Mediums zu erfassen, unterschiedliche Medien, beispielsweise unterschiedliche Kraftstoffarten wie Dieselkraftstoff oder Biokraftstoffe wie beispielsweise Rapsölmethylester können durch einen derartigen Sensor nicht unterschieden werden. Darüber hinaus kann ein derartiger Sensor keine Aussage über die Art oder die Qualität eines Mediums machen.

Es sind auf kapazitive Mediensensoren bekannt, die jedoch mit hohen Frequenzen und mit Brückenschaltungen arbeiten, die bezüglich der elektromagnetischen Verträglichkeit sehr störanfällig sind und zudem als Sender wirken können.

Die der Erfindung zu Grunde liegende Aufgabe besteht daher darin, einen Mediensensor der eingangs genannten Art zu schaffen, der für verschiedene Medien insbesondere verschiedene Kraftstoffarten und Wasser empfindlich ist.

Diese Aufgabe wird gemäß der Erfindung durch die Ausbildung gelöst, die im Patentanspruch 1 angegeben ist.

Bevorzugte Ausgestaltungen und Weiterbildungen des erfindungsgemäßen Mediensensors sind Gegenstand der Patentansprüche 2 bis 6.

Da der erfindungsgemäße Mediensensor so ausgebildet ist, dass sein Kondensator ein homogenes elektrisches Feld erzeugt, liefert er ein Messergebnis, das für die Unterscheidung von verschiedenen Kraftstoffarten exakt auswertbar ist. Streufelder, die bei kapazitiven Sensoren mit nicht homogenen Feldern, wie beispielsweise Sensoren mit Platten- oder Stabelektroden auftreten, können das Messergebnis nicht negativ beeinflussen.

Der erfindungsgemäße Mediensensor wird so betrieben, dass sein Kondensator mit einer niederfrequenten Taktrate mit einer Gleichspannung aufgeladen wird, der zeitliche Anstieg der Ladespannung des Kondensators in Impulse umgewandelt wird, deren Dauer zur Kapazität der Kondensators proportional ist, und die Impulse in eine sich mit der Kapazität ändernden Signalspannung umgewandelt werden.

Mit dem erfindungsgemäßen Mediensensor ist es in dieser Weise zum einen möglich, die Beschaffenheit eines bestimmten Medium beispielsweise eines verwendeten Kraftstoffs zu bestimmen und zu kennen, um die künftigen Abgasvorschriften einhalten zu können, was insbesondere für mit Dieselkraftstoff oder Biodieselkraftstoff betriebene Fahrzeuge gilt, und kann darüber hinaus das Einspritzsystem vor einer Korrosion geschützt werden, da eine Warnung bewirkt werden kann, wenn im Kraftstofffilter ein bestimmter Wasserstand erreicht ist. Beide Funktionen sind mit dem erfindungsgemäßen Sensor möglich.

Im Folgenden werden anhand der zugehörigen Zeichnung besonders bevorzugte Ausführungsbeispiele der Erfindung näher beschrieben. Es zeigen
Fig. 1 eine Teilschnittansicht des Ausführungsbeispiels,
Fig. 2 eine perspektivische Ansicht des in Fig. 1 dargestellten Ausführungsbeispiels mit aufgebrochener äußerer Elektrode und
Fig. 3 eine perspektivische Ansicht eines weiteren Ausführungsbeispiels des erfindungsgemäßen Mediensensors mit zusätzlicher Mittelelektrode.

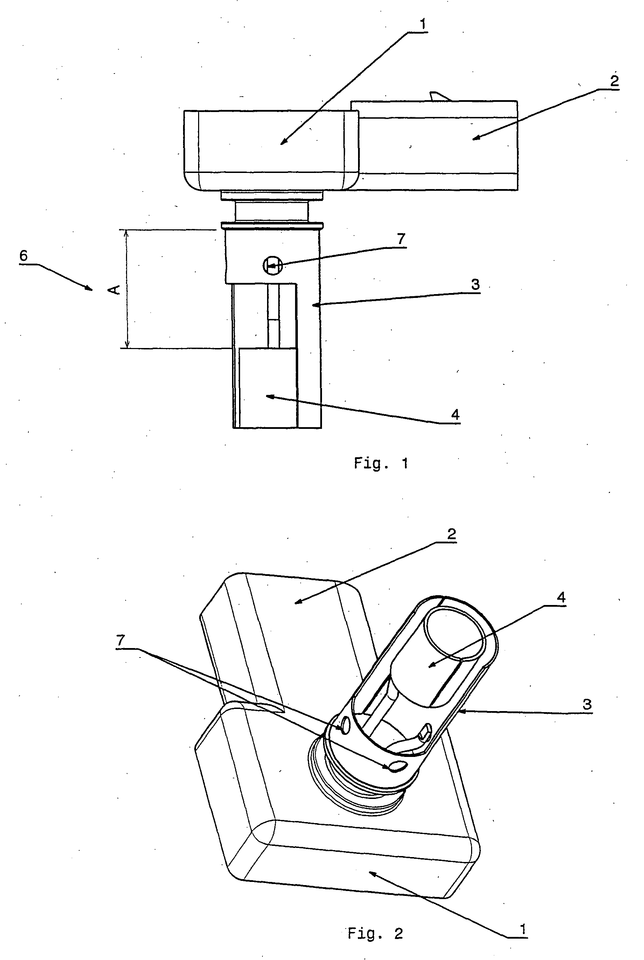

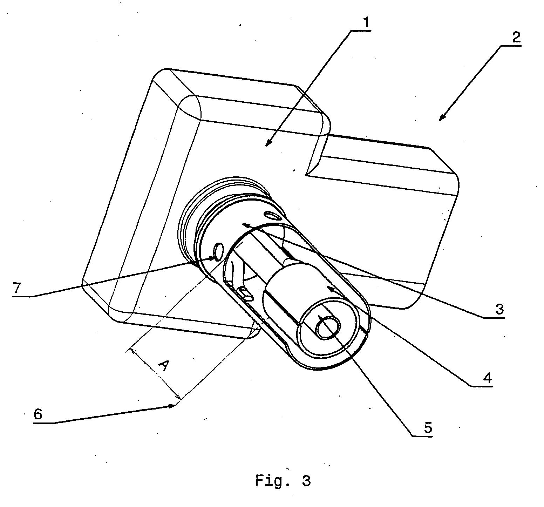

Das in den Figuren 1 und 2 dargestellte Ausführungsbeispiel des erfindungsgemäßen Mediensensors umfasst ein Gehäuse 1, an dem ein Kondensator angeordnet ist, der aus einer Außenelektrode 3 und einer Innenelektrode 4 besteht. Beide Elektroden sind über Anschlussleiter jeweils mit einem elektrischen Anschluss 2 verbunden.

Der Kondensator besteht aus Hohlkörperelektroden 3, 4, die koaxial angeordnet sind, und insbesondere aus zwei zylindrischen Elektroden, wobei in der äußeren Elektrode 3 Bohrungen 7 ausgebildet sind, die die Durchflutung des Sensors mit dem zu erfassenden Medium unterstützen.

Ein derartiger Mediensensor wird beispielsweise von unten in den Kraftstofffilter eines Kraftfahrzeuges eingebaut, um das Überschreiten der zulässigen Wassermenge im Filter zu erfassen. Der Sensor kann jedoch auch in einer beliebigen Lage im Kraftstofftank eingesetzt werden.

Die zulässige Wassermenge wird dann durch den Höhenabstand 6 der Innenelektrode 4 des Kondensators definiert. Sobald der Wasserstand so weit angestiegen ist, dass sich zwischen den Elektroden 3 und 4 Wasser befindet, ändert sich die Dielektrizitätskonstante so stark, dass das über eine entsprechende Auswerteschaltung sicher erfasst werden kann.

Die Bohrungen 7, die das Durchfluten des Sensors unterstützen, stellen sicher, dass die Kraftstoffart oder der Wasserstand im Inneren des Sensors auch der Kraftstoffart oder dem Wasserstand außerhalb des Sensors entspricht.

Die Elektroden 3, 4 sind bei dem in Fig. 1 und 2 dargestellten Ausführungsbeispiel als koaxiale Zylinderelektroden vorgesehen, sie können aber auch einen ovalen oder einen rechteckigen insbesondere quadratischen Querschnitt haben, so lange sie ein homogenes elektrisches Feld liefern und Einwirkungen von Streufeldern vermieden sind.

Wenn die mittlere Elektrode 4 mit einer Gleichspannung insbesondere Gleichspannungsimpulsen beaufschlagt wird, wirkt darüber hinaus die äußere Elektrode 3 als Abschirmung, was zur Einhaltung der EMV-Grenzwerte dient.

Der oben beschriebene Mediensensor arbeitet in der folgenden Weise:

Im Ruhezustand liegt die Mittelelektrode 4 niederohmig auf Massepotential, so dass beide Elektroden 3, 4 in diesem Zustand spannungsfrei sind.

Zu Beginn eines Grundtaktes wird die Mittelelektrode 4 hochohmig geschaltet und wird mit dem Aufladen des Kondensators begonnen. Der Ladevorgang des Kondensators endet, wenn die Spannung an der Mittelelektrode 4 einen bestimmten Wert erreicht, der als Schwellenwert bezeichnet werden kann. Gleichzeitig mit dem Ende des Ladungsvorgangs wird die Mittelelektrode 4 wieder niederohmig auf Massepotential gelegt. Dadurch wird der Kondensator entladen.

Das Ausgangssignal des Kondensators liegt an einem Multivibrator, der einen Impuls erzeugt, dessen Länge in einem linearen Zusammenhang mit der Sensorkapazität steht. Zwischen der Sensorkapazität und der Impulsdauer besteht ein linearer Zusammenhang.

Das Ausgangssingal des Multivibrators kann über ein Tiefpassfilter in eine Signalspannung umgewandelt werden, die nur noch in den gewünschten Spannungsbereich durch Verstärkung oder Offset-Korrektur umgewandelt werden muss und zur Anzeige verwandt werden kann.

Zur Durchführung dieses Verfahrens wird eine Schaltungsanordnung benötigt, die einen Taktgenerator aufweist, dessen Taktausgangssignal an dem Multivibrator liegt, der seinerseits einen Signaleingang aufweist, der mit der Mittelelektrode 4 des Mediensensors verbunden ist, an dem die Ladespannung liegt. Über diesen Signaleingang wird die Elektrode 4 des Mediensensors hochohmig und niederohmig geschaltet wobei das Tiefpassfilter, an dem das Ausgangssignal des Multivibrators liegt an seinem Ausgang die gewünschte Signalspannung liefert, die zur Kapazität des Mediensensors proportional ist.

Wenn der Kondensator mit einer niederfrequenten Taktrate mit einer Gleichspannung aufgeladen wird und der zeitliche Anstieg der Ladespannung des Kondensators in Impulse umgewandelt wird, deren Dauer zur Kapazität des Kondensators proportional ist ergibt sich eine Signalspannung, die sich linear mit der Kapazität des Kondensators ändert und damit eine Anzeige für das entsprechende Medium liefert, das sich zwischen den Kondensatorelektroden 3, 4 befindet.

Fig. 3 zeigt ein weiteres Ausführungsbeispiel des erfindungsgemäßen Mediensensors, das sich von dem in den Fig. 1 und 2 dargestellten Ausführungsbeispiel dadurch unterscheidet, dass eine zusätzliche Mittelelektrode 5 vorgesehen ist, die elektrisch mit der Außenelektrode 3 verbunden ist, wobei alle Elektroden 3, 4, 5 koaxial angeordnet und ausgebildet sind. Diese zusätzliche Mittelelektrode 5 erhöht die Fläche des Kondensators, was ein sicheres Erkennen verschiedener Kraftstoffarten gewährleistet und es erlaubt, die Bauhöhe des Sensors zu begrenzen.

Mit dem erfindungsgemäßen Mediensensor ist es möglich, mit großer Sicherheit Wasser in Kraftstofffiltern zu erkennen und in Kombination mit der Wassererkennung die Kraftstoffart zu erkennen, wobei eine ausgezeichnete Durchspülung des Sensors und eine gute Abschirmung durch die äußere Elektrode gewährleistet sind.

## Patentansprüche

1. Mediensensor zum Erfassen eines Mediums und/oder zum Bestimmen der Beschaffenheit eines Mediums mit einem Gehäuse, an dem ein Kondensator angeordnet ist, zwischen dessen Elektroden ein Medium eingeführt werden kann, **dadurch gekennzeichnet, dass** die Kondensatorelektroden koaxial angeordnete Hohlkörperelektroden sind, die ein homogenes elektrisches Feld erzeugen.

2. Mediensensor nach Anspruch 1, **dadurch gekennzeichnet, dass** der Kondensator aus zylindrischen Elektroden aufgebaut ist.

3. Mediensensor nach Anspruch 1, **dadurch gekennzeichnet, dass** der Kondensator aus im Querschnitt ovalen Elektroden aufgebaut ist.

4. Mediensensor nach Anspruch 1, **dadurch gekennzeichnet, dass** der Kondensator aus im Querschnitt rechtwinkligen Elektroden aufgebaut ist.

5. Mediensensor nach einem der vorhergehenden Ansprüche, **gekennzeichnet durch** eine zusätzliche koaxiale Innenelektrode, die mit der Außenelektrode des Kondensators elektrisch verbunden ist.

6. Mediensensor nach einem der vorhergehenden Ansprüche, **dadurch gekennzeichnet, dass** in der Außenelektrode Bohrungen zum Durchspülen des Kondensators mit einem Medium vorgesehen sind.
